# FASCICULE DE BREVET EUROPEEN

(11) **EP 3 858 492 B1**
(45) Date de publication et mention de la délivrance du brevet: **15.05.2024**
(21) Numéro de dépôt: 21152163.8
(22) Date de dépôt: 18.01.2021
(51) Int. Cl.: B05B 11/10, B05B 12/14, A45D 40/24, A61M 35/00, A45D 34/04, A45D 44/00, B01F 33/841, A45D 34/00

(54) **DISPOSITIF DE DISTRIBUTION D'UNE FORMULATION D'AU MOINS DEUX COMPOSES CHOISIS PARMI UN ENSEMBLE DE COMPOSES SELECTONNABLES ET CONTENANT ASSOCIE**
VORRICHTUNG ZUR ABGABE EINER FORMULIERUNG VON MINDESTENS ZWEI VERBINDUNGEN, DIE AUS EINEM SATZ VON AUSWÄHLBAREN VERBINDUNGEN AUSGEWÄHLT WIRD, UND ENTSPRECHENDER BEHÄLTER
DEVICE FOR DISPENSING A FORMULATION OF AT LEAST TWO COMPOUNDS CHOSEN FROM A GROUP OF SELECTABLE COMPOUNDS AND ASSOCIATED CONTAINER

(30) Priorité: 31.01.2020 FR 2000948
(43) Date de publication de la demande: 04.08.2021
(73) Titulaire: IEVA, 75002 Paris (FR)
(72) Inventeur: CLAPPAZ, Cyril, 38780 Eyzin-Pinet (FR); MOULINIER, David, 69006 Lyon (FR)
(74) Mandataire: Cabinet Laurent & Charras

(56) Documents cités:
- WO-A1-2012/018999
- WO-A1-2018/169823
- WO-A1-2018/211253
- KR-Y1- 200 440 663
- US-A1- 2019 201 926
- US-A1- 2020 002 152

## Description

### Domaine technique

L'invention concerne un dispositif de distribution d'une formulation d'au moins deux composés choisis parmi un ensemble de composés sélectionnables.

Les différents contenants intégrant ces composés sélectionnables peuvent se présenter sous la forme de contenants interchangeables permettant un réapprovisionnement des composés sélectionnables du dispositif de distribution. Ainsi, l'invention concerne également un contenant associé au dispositif de distribution.

L'invention peut être mise en oeuvre dans un grand nombre de domaines pour lesquels il est recherché d'obtenir une formulation à partir de différents composés possibles.

L'invention trouve une application particulièrement avantageuse dans le domaine des cosmétiques dans lequel il est recherché de pouvoir formuler une crème s'adaptant sur mesure aux besoins de traitement de la peau d'un utilisateur.

### Etat de la technique

Les produits cosmétiques ont évolué ces dernières années pour répondre spécifiquement aux besoins de traitement de la peau d'un utilisateur. Pour ce faire, plusieurs solutions proposent désormais de tester les caractéristiques physiologiques de la peau d'un utilisateur afin de le guider vers l'achat d'une crème adaptée pour ses besoins. Au lieu de conseiller l'achat d'une crème standard, la société IOMA^{®} propose une solution nommée « *IOMA In.Lab* » permettant de tester les paramètres physiologiques d'un utilisateur dans un magasin afin de formuler une crème sur mesure.

Cependant, les paramètres physiologiques de la peau d'un utilisateur peuvent varier au cours du temps et il est recherché de pouvoir formuler des traitements sur-mesure qui évoluent dans le temps en fonction des sollicitations subies par la peau de l'utilisateur. Pour ce faire, la société IEVA^{®} propose un bijoux « *Twin.C* » destiné à être porté par l'utilisateur pour mesurer, au cours du temps, les paramètres du milieu dans lequel l'utilisateur évolue. Ce bijoux « *Twin.C »* permet de mesurer les pollutions extérieure ou intérieure, l'exposition au soleil, le bruit ambiant, la luminosité, la température et l'humidité, et le nombre de pas. Avec toutes ces informations, il est possible de conseiller un rituel santé/bien-être adapté à chaque utilisateur et capable d'évoluer au cours du temps. En outre, il est possible de déterminer la formulation d'une crème spécifiquement adaptée aux besoins d'un utilisateur.

La réalisation de cette crème peut ensuite être formulée en magasin, par exemple avec la solution « *IOMA In.Lab* ». Pour être plus indépendant et réactif, cette crème peut également être réalisée à domicile. Pour ce faire, plusieurs sociétés proposent des machines de formulation d'un produit cosmétique sur mesure, par exemple le formulateur « *HyLab* » de la société Romy^{®} ou la solution « *Custom D.O.S.E* » de la société L'Oréal^{®} dont la machine est décrite dans le brevet WO 2017/95854.

Pour générer sa crème sur mesure, l'utilisateur dispose d'un certain nombre de capsules contenant les composés des différentes crèmes qu'il peut formuler à partir de la machine. Lors de la fabrication de la crème, la machine procède à l'extraction des quantités recherchées des composés présents dans les différentes capsules insérées dans la machine. La machine mélange ensuite les composés extraits pour obtenir une crème dont l'aspect correspond à une crème standard mais dont les composés et leurs quantités sont déterminés sur mesure.

Ce type de solution est néanmoins très peu transportable car, à l'instar d'une machine à café, cette machine nécessite une alimentation électrique et un grand nombre d'actionneurs électromécaniques pour obtenir la formulation recherchée, et notamment pour procéder au mélange des composés.

En outre, l'utilisation de capsules pour intégrer les différents composés pouvant être utilisés pour obtenir la formulation entraîne des contraintes de stockage et de pollution générée par les rebus des capsules usagées.

Le problème technique de l'invention consiste à obtenir une formulation d'au moins deux composés choisis parmi un ensemble de composés sélectionnables de manière beaucoup plus transportable que les solutions existantes.

### Description de l'invention

Pour résoudre ce problème technique, l'invention propose un dispositif de distribution permettant d'extraire des composés stockés dans des contenants pour les acheminer sur une surface de distribution. Ainsi, au lieu de réaliser complètement une crème avec l'étape nécessaire de mélange, l'invention propose de délivrer une quantité recherchée de la formulation pour chaque application.

En délivrant les composés sur la surface de distribution, l'utilisateur peut alors récupérer les composés avec son doigt pour appliquer la formulation sur sa peau. Le doigt de l'utilisateur récupérant les composants effectue ainsi l'association des composés.

L'invention est donc issue d'une découverte selon laquelle les principes actifs d'une crème ne découlent pas de l'étape de mélange classiquement effectuée mais des composés pris indépendamment. Ainsi, une simple association des composés, réalisée avec le doigt d'un utilisateur, suffit à obtenir les mêmes effets sur la peau.

A cet effet, selon un premier aspect, l'invention concerne un dispositif de distribution d'une formulation d'au moins deux composés choisis parmi un ensemble de composés sélectionnables, ledit dispositif comportant :
- une pluralité de contenants stockant lesdits différents composés sélectionnables ;
- des moyens de pompage associés à chaque contenant ;
- une surface de distribution comportant au moins deux orifices débouchant destinés à acheminer au moins deux composés choisis ;
- des moyens de sélection permettant de disposer lesdits au moins deux orifices débouchant en face des moyens de pompage desdits composés choisis de sorte à permettre auxdits moyens de pompage desdits composés choisis d'acheminer lesdits composés choisis sur la surface de distribution; et
- un actionneur configuré pour n'activer que les moyens de pompage desdits composés choisis et ne délivrer que lesdits au moins deux composés choisis sur ladite surface de distribution.

L'invention permet ainsi de délivrer, sur une surface de distribution, une formulation spécifique obtenue à partir d'un ensemble de composés sélectionnables. Ce faisant, l'invention permet à un utilisateur de réaliser une dose d'une crème adaptée à ses besoins.

Contrairement aux machines de formulation sur mesure existantes qui visent à fabriquer un pot de crème complet, l'invention propose de distribuer uniquement une ou plusieurs doses nécessaires pour une application, si bien que le dispositif de l'invention peut être beaucoup plus compact et transportable que les machines de formulation existantes.

Selon l'invention, les moyens de pompage peuvent correspondre à des pompes actionnées mécaniquement ou électromécaniquement. Dans le cas de pompes électromécaniques, le dispositif de distribution intègre préférentiellement des batteries et une carte de contrôle des actionneurs électromécaniques intégrés dans le dispositif de distribution de sorte à former un dispositif de distribution autonome et compact.

De préférence, le dispositif de distribution comporte des pompes mécaniques pour former les moyens de pompage de sorte à obtenir un dispositif de distribution purement mécanique. Dans ce mode de réalisation, ledit actionneur correspond préférentiellement à un bouton poussoir dont la face supérieure correspond à ladite surface de distribution, lesdits moyens de pompage ayant une course supérieure ou égale à la course du bouton poussoir. Ainsi, l'activation du bouton poussoir active les pompes et permet de délivrer les composés sélectionnés sur la surface de distribution.

La sélection des composés peut être réalisée par tous moyens mécaniques ou électromécaniques. De préférence, les moyens de sélection correspondent à des moyens mécaniques qui permettent de déplacer les orifices ou les contenants pour modifier la correspondance entre les orifices et les contenants.

Selon un premier mode de réalisation dans lequel les orifices sont fixes, au moins un desdits moyens de sélection correspond à un barillet intégrant plusieurs contenants, une rotation dudit barillet entraînant une modification de la correspondance entre un orifice et l'un des contenants intégrés dans ledit barillet.

Ce mode de réalisation permet de sélectionner mécaniquement les composés choisis pour distribuer la formulation. Lorsque les composés sont sélectionnés, l'utilisateur peut alors simplement activer les moyens de pompage via l'actionneur pour obtenir les composés de la formulation sur la surface de distribution.

Il est possible d'inhiber le fonctionnement de l'actionneur pour éviter d'éventuelles fausses manipulations du dispositif de distribution. Pour ce faire, il est possible d'utiliser un bouchon de protection qui, une fois en place, bloque l'accès à l'actionneur. Selon ce mode de réalisation, ledit dispositif comporte un bouchon de protection amovible configuré pour recouvrir ladite surface de distribution et inhiber l'activation dudit actionneur.

En ce qui concerne les composés utilisés par le dispositif de distribution, ils peuvent varier en fonction des applications du dispositif de distribution. De préférence, ledit dispositif comporte deux types de composés distincts stockés dans deux types de contenants distincts ; lesdits au moins deux composés étant choisis pour obtenir une formulation d'au moins un premier type de composés et d'au moins un second type de composés.

Par exemple, ledit dispositif comporte entre un et trois composés du premier type et entre six et huit composés du second type de sorte à permettre l'obtention d'un grand nombre de formulations possibles.

En fonction des applications, des composés de natures distinctes peuvent être utilisés. Par exemple, l'invention peut être mise en oeuvre pour la formulation d'une crème cosmétique. Dans ce mode de réalisation, ledit premier type de composés peut correspondre à des bases, par exemple des bases aqueuses ou huileuses, et ledit second type de composés peut correspondre à des sérums.

Pour une crème cosmétique, une formulation comporte classiquement une base et un sérum avec une quantité plus importante de base que de sérum. Pour obtenir cette différence de volume avec le dispositif de distribution, les premiers contenants présentent un volume supérieur à des seconds contenants et lesdits moyens de pompage associés auxdits premiers contenants présentent un volume de pompage plus important que lesdits moyens de pompage associés auxdits seconds contenants pour une même activation dudit actionneur.

De préférence, ladite pluralité de contenants est intégrée dans au moins un support présentant au moins deux parties amovibles de sorte à permettre un remplacement ou un remplissage d'au moins un contenant. En remplaçant les contenants selon ses besoins, un utilisateur peut augmenter le nombre de formulations possibles qu'il peut réaliser avec ledit dispositif de distribution de l'invention en utilisant des contenants avec d'autres composés que ceux présents dans le dispositif de distribution.

En outre, le remplacement des contenants permet également d'augmenter la durée de vie du dispositif de distribution en remplaçant ou en remplissant les contenants vides. La possibilité de pouvoir remplir les contenants vidés limite également les rebus générés par le dispositif de distribution de l'invention améliorant donc son empreinte écologique.

### Description sommaire des figures

La manière de réaliser l'invention ainsi que les avantages qui en découlent, ressortiront bien des modes de réalisation qui suivent, donnés à titre indicatif mais non limitatif, à l'appui des figures annexées dans lesquelles les figures 1 à 16 représentent :
La figure 1 est une vue de face d'un dispositif de distribution d'une formulation selon un premier mode de réalisation de l'invention qui n'est pas inclus dans l'objet des revendications ;
La figure 2 est une vue en perspective éclatée du dispositif de distribution de la figure 1 ;
La figure 3 est une vue en coupe du dispositif de distribution de la figure 1 ;
La figure 4 est une vue de dessus d'une bague inférieure du dispositif de distribution de la figure 1 ;
La figure 5 est une vue en perspective de la bague inférieure de la figure 4 ;
La figure 6 est une vue en perspective d'une bague supérieure du dispositif de distribution de la figure 1 ;
La figure 7 est une vue en coupe partielle du dispositif de distribution de la figure 1 ;
La figure 8 est une vue en perspective d'un contenant adapté au dispositif de distribution de la figure 1 ;
La figure 9 est une vue de face d'un dispositif de distribution d'une formulation selon un second mode de réalisation de l'invention ;
La figure 10 est une vue en perspective éclatée du dispositif de distribution de la figure 9 ;
La figure 11 est une vue en coupe du dispositif de distribution de la figure 9 ;
La figure 12 est une vue en perspective d'un barillet inférieur du dispositif de distribution de la figure 9 ;
La figure 13 est une vue en perspective d'un barillet supérieur du dispositif de distribution de

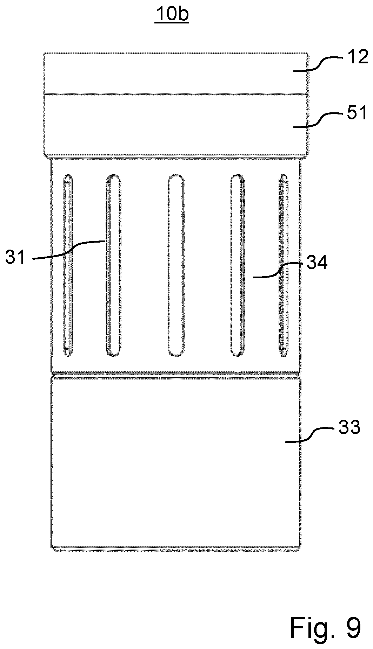

la figure 9 ;

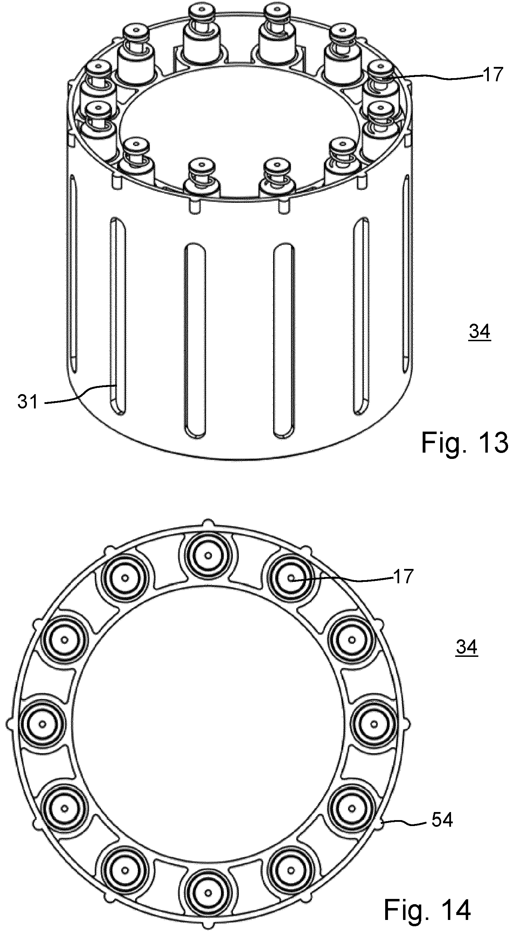

La figure 14 est une vue de dessus du barillet supérieur de la figure 13 ;

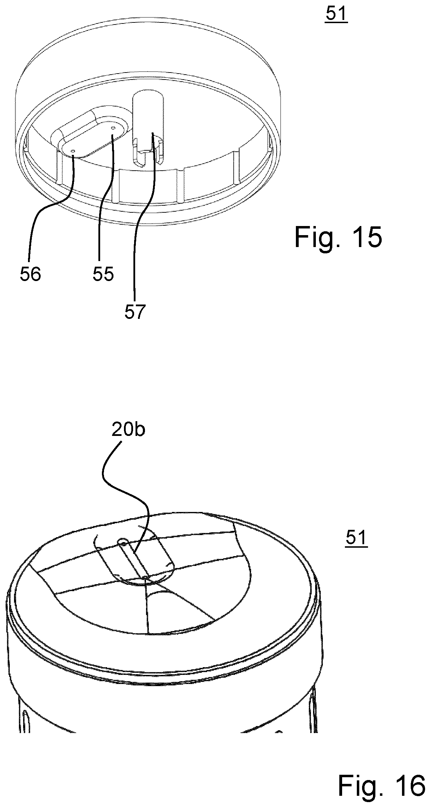

La figure 15 est une vue en perspective de dessous de la surface de distribution du dispositif de distribution de la figure 9 ; et
La figure 16 est une vue en perspective de dessus de la surface de distribution du dispositif de distribution de la figure 9.

### Description détaillée de l'invention

Dans la description qui suit, deux modes de réalisation distincts illustrent un dispositif de distribution entièrement mécanique. En variante, l'invention peut être mise en oeuvre avec des composants électromécaniques.

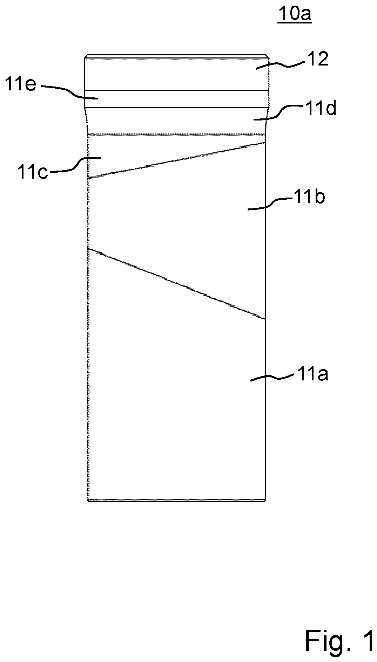

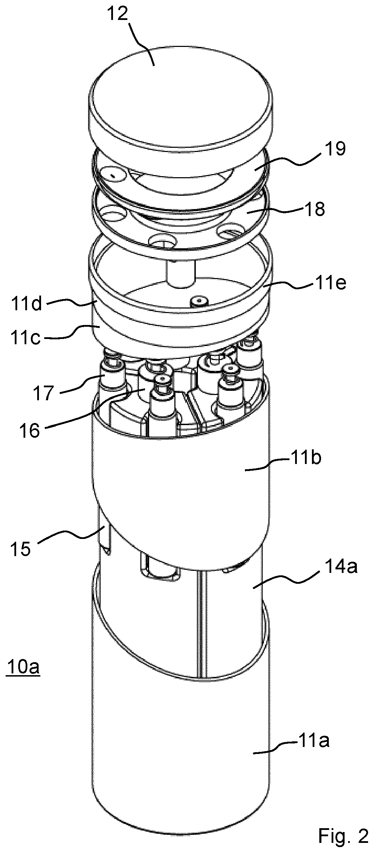

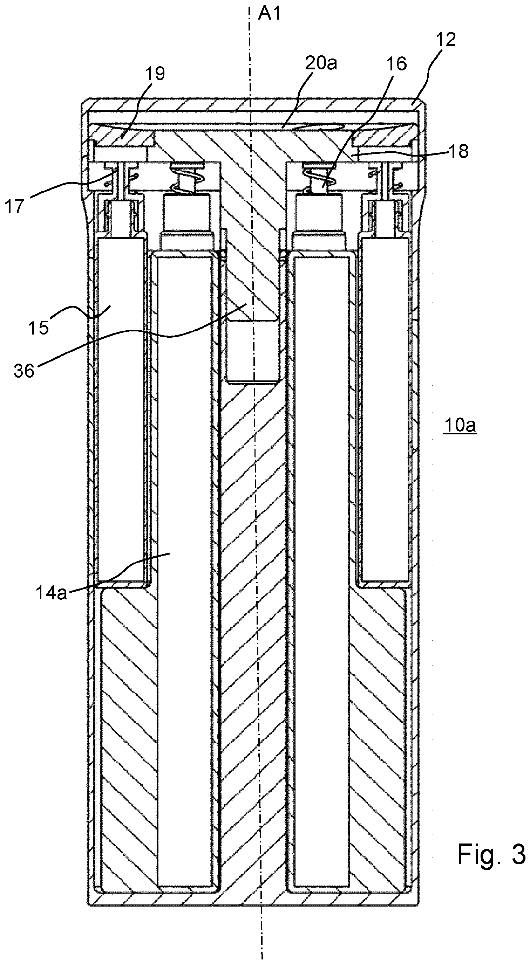

Les figures 1 à 3 illustrent un dispositif de distribution **10a** selon un premier mode de réalisation de l'invention qui n'est pas inclus dans l'objet des revendications. Dans ce mode de réalisation, le dispositif de distribution **10a** présente une forme sensiblement cylindrique avec une hauteur comprise entre 7 et 15 cm et un diamètre compris entre 3 et 10 cm.

Ainsi, le dispositif de distribution **10a** se présente sous la forme d'un flacon transportable.

Ce dispositif de distribution **10a** présente une coque externe formée par plusieurs parties superposées **11a-11e** et fixées entre elles. Les trois parties inférieures **11a** à **11c** présentent des formes cylindriques tronquées complémentaires permettant d'obtenir un cylindre intégrant les différents composés sélectionnables. L'assemblage de ces trois parties inférieures **11a** à **11c** permet de varier les matières, les textures ou les couleurs de ce cylindre. Au-dessus de ce cylindre, la coque présente un épaulement **11d** surmonté par un rebord **11e** cylindrique. En outre, ces différentes parties **11a-11e** de la coque sont montés de manière amovible de sorte à pour ouvrir la coque et remplacer ou remplir un des contenants **14a, 15.** L'épaulement **11d** et le rebord **11e** permettent d'intégrer les moyens de pompage **16-17** et les moyens de sélection **18-19.**

En outre, le rebord **11e** est configuré pour supporter un bouchon de protection **12** permettant de fermer le dispositif de distribution **10a.** Par exemple, le bouchon de protection **12** peut-être monté par clipsage ou par vissage sur le rebord **11e.**

À l'intérieur du dispositif de distribution **10a,** les différents composés sélectionnables sont intégrés dans des contenants **14a-15.** Les composés sélectionnables peuvent être répartis dans des contenants de fort volume **14a** intégrant des composés de base et des contenants de faible volume **15** intégrant des sérums permettant de formuler une crème cosmétique. Par exemple, les contenants **14a** présentent un volume compris entre 20 et 35 ml alors que les contenants **15** présentent un volume compris entre 3 et 8 ml.

Bien entendu, d'autres types de composés peuvent être utilisés pour obtenir d'autres formulations sans changer invention.

L'extraction des composés stockés dans ces contenants **14a-15** est préférentiellement réalisée par une pompe manuelle **16** ou **17** associée à chaque contenant **14a-15.**

Une pompe manuelle peut aspirer les composés à travers un tuyau d'alimentation s'étendant jusqu'au fond du contenant. De préférence, un piston est intégré dans chaque contenant **14a-15** de sorte qu'une pression effectuée sur sa pompe **16-17** entraine une dépression qui tend à déplacer le piston vers le sommet du contenant pour extraire le composé à travers une ouverture ménagée au sommet de la pompe **16-17.**

Pour obtenir la formulation recherchée, le dispositif de distribution **10a** permet d'acheminer les composés choisis sur une surface de distribution **20a** afin qu'un utilisateur puisse la collecter avec son doigt. Pour former les moyens de sélection, le mode de réalisation des figures 1 à 8 propose d'utiliser deux bagues concentriques **18-19** formant à la fois la surface de distribution **20a,** les moyens de sélection des composés choisis et un actionneur pour les pompes **16-17.** La bague **19** étant montée sur la bague **18,** la bague **18** est appelée « bague inférieure » alors que la bague **19** est appelée « bague supérieure » dans la suite de la description.

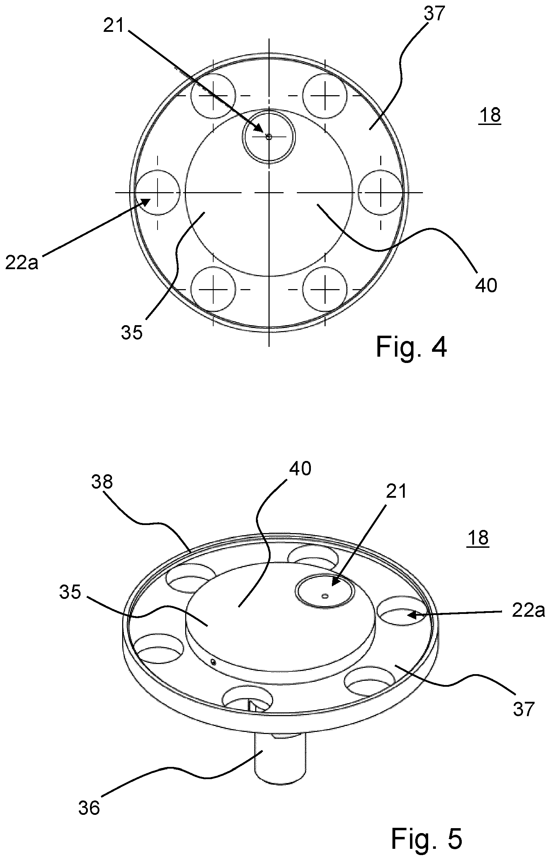

Tel qu'illustré sur les figures 4 et 5, la bague inférieure **18** présente un cylindre central **35** dont la face inférieure est pourvue d'un pion de guidage **36.** Par exemple, le cylindre central **35** peut présenter une épaisseur comprise entre 0.8 et 5mm et un diamètre compris entre 2 et 8 cm. La face inférieure de la bague inférieure **18** s'étend sous la forme d'un disque **37** dont le diamètre est supérieur à celui du cylindre central **35.** Par exemple, le disque **37** peut présenter une épaisseur comprise entre 0.4 et 2mm et un diamètre compris entre 3 et 10 cm. La partie terminale de ce disque **37** présente un rebord **38** s'étendant orthoradialement en direction de la face supérieure **40** du cylindre central **35.** La hauteur de ce rebord **38** peut être comprise entre 0.2 et 2 mm.

Le cylindre central **35** présente un orifice cylindrique **21** débouchant sur les deux faces supérieure **40** et inférieure et réalisé proche des bords du cylindre central **35.** Cet orifice cylindrique **21** peut présenter un diamètre compris entre 0.4 et 2 mm. En outre, six autres orifices **22a** sont régulièrement répartis dans le disque **37.** Ces autres orifices cylindriques **22a** peuvent également présenter un diamètre compris entre 0.4 et 2 mm.

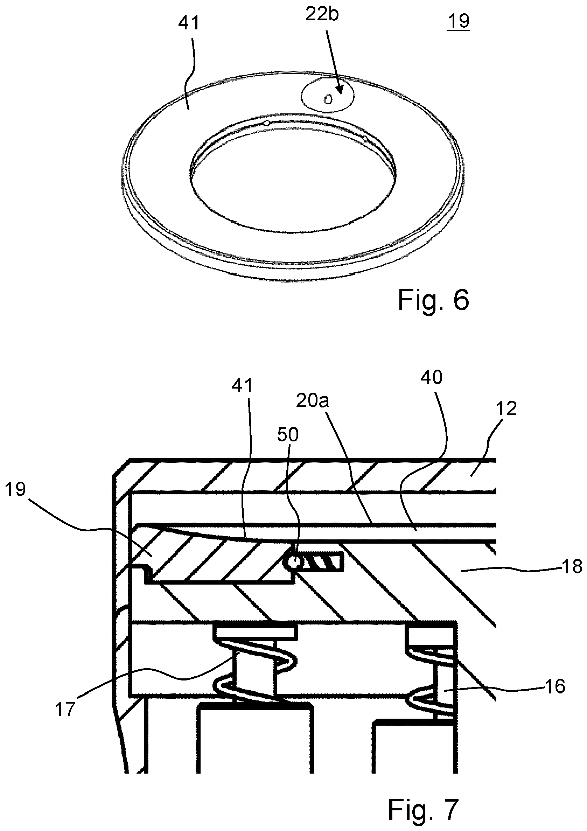

Tel qu'illustré sur la figure 6, la bague supérieure **19** se présente sous la forme d'un anneau **19.** Cet anneau **19** peut présenter un diamètre interne compris entre 2 et 8 cm et un diamètre externe compris entre 3 et 10 cm. L'épaisseur de cet anneau **19** est préférentiellement comprise entre 0.4 et 2 mm. Un orifice cylindrique débouchant **22b** est ménagé dans l'épaisseur de cet anneau **19.** Cet orifice **22b** peut présenter un diamètre compris entre 0.4 et 2 mm.

Tel qu'illustré sur la figure 7, la surface de distribution **20a** est obtenue par les deux surfaces supérieures **40-41** des deux bagues **18-19.** Ces deux surfaces supérieures **40-41** sont sensiblement coplanaires lorsque la bague supérieure **19** est montée autour de la bague inférieure **18.** Ainsi, le centre de la surface de distribution **20a** est formé par la face supérieure **40** de la bague inférieure **18** passant à travers la bague supérieure **19** alors que le pourtour de cette surface de distribution **20a** est formé par la face supérieure **41** de la bague inférieure **18.**

Pour sélectionner les composés et les acheminer sur la surface de distribution **20a,** les deux bagues **18-19** sont mobiles en rotation autour d'un axe central **A1.** De préférence, ces bagues **18-19** ne peuvent être entraînées en rotation que lorsque le bouchon de protection **12** est retiré du dispositif de distribution **10a.**

La bague inférieure **18** est montée directement au-dessus de l'ensemble des pompes **16-17** et son orifice débouchant **21** permet d'acheminer un composé présent dans un contenant **14a** sur sa face supérieure **40,** c'est-à-dire sur la surface de distribution **20a.** L'acheminement d'un composé présent dans un contenant **15** est réalisé en traversant les orifices **22a** de la bague inférieure **18** et l'orifice **22b** de la bague supérieure **19.**

L'activation des pompes **16-17** est obtenue par le déplacement des deux bagues **18-19.** Pour ce faire, les deux bagues **18-19** sont posées sur l'ensemble des pompes **16-17** et le pion **36** assure le guidage du déplacement des deux bagues **18-19.**

En outre, la bague supérieure **19** est cintrée sur la bague inférieure **18** au niveau du rebord **38** de sorte que les deux bagues **18-19** soient mobiles conjointement selon l'axe **A1.** Le déplacement des deux bagues **18-19** est consécutif à un appui d'un utilisateur sur la surface de distribution **20a.** Cet appui a pour effet de compresser toutes les pompes **16-17** et d'entraîner une distribution de la formulation des composés sélectionnés. En effet, lors de l'activation des pompes **16-17,** seules les pompes disposées en face des orifices **21** et **22b** voient leurs composés acheminés sur la surface de distribution **20a.** Après l'activation, c'est-à-dire après l'appui de l'utilisateur, les deux bagues **18-19** sont repositionnées à leurs positions initiales par la décompression des ressorts des pompes **16-17.**

Pour faciliter la sélection des différents composés et éviter qu'un des orifices **21** et **22b** ne se retrouve disposé entre deux pompes **16-17,** des détrompeurs de positions sont préférentiellement mis en oeuvre. Par exemple, la figure 7 illustre un pion **50** et un ressort intégrés dans un alésage de la bague inférieure **18** et configurés pour coopérer avec des alésages présents à chaque position possible de la bague supérieure **19.** Ainsi, l'utilisateur peut ressentir une différence d'effort de rotation de la bague supérieure **19** entre les positions possibles et les positions intermédiaires de la bague **19.**

Ce mode de réalisation, illustré sur les figures 1 à 7, qui n'est pas inclus dans l'objet des revendications, peut être mis en oeuvre quel que soit la forme des contenants. Par exemple, l'invention peut être mise en oeuvre avec des contenants standard présentant une forme cylindrique.

De préférence, pour optimiser l'espace à l'intérieur de la coque **11a-11e,** le contenant de fort volume **14a** présente une forme spécifique permettant à la fois de contenir un composé et d'intégrer des logements **30** pour positionner des contenants cylindriques **15** de plus faible volume.

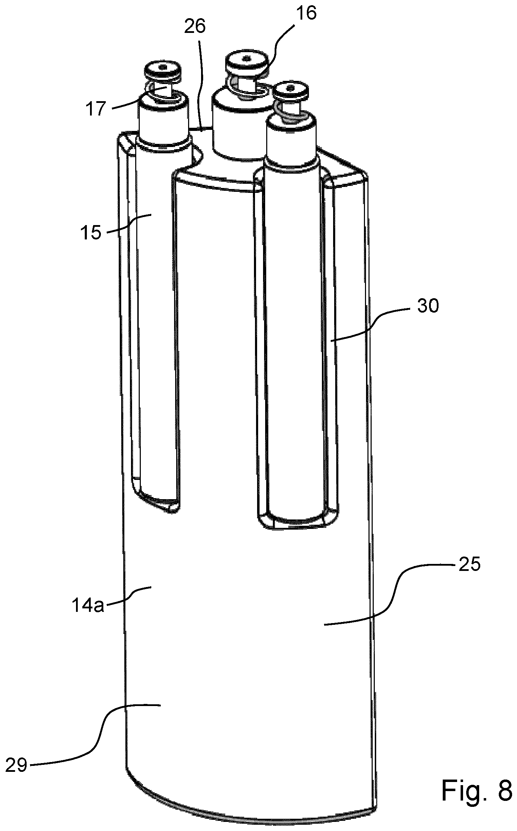

Tel qu'illustré sur la figure 8, le contenant **14a** présente un corps creux **25** présentant sensiblement la forme d'une portion de cylindre avec deux faces planes et une face hémisphérique **29** s'étendant dans la hauteur de la portion de cylindre.

La face inférieure du corps **25** présente une surface sensiblement plane alors que la face supérieure **26** du corps **25** intègre une pompe **16** permettant d'extraire le composé stocké dans le corps **25.** En outre, la face hémisphérique **29** présente deux logements **30** débouchant sur la face supérieure **26** s'étendant sensiblement entre la moitié de la hauteur du corps **25** et la surface supérieure **26.**

Outre l'utilisation des bagues **18-19** pour sélectionner les composés de la formulation, il est également possible d'utiliser un ou plusieurs barillets **33-34** en remplacement d'une des bagues **18-19.** En outre, il est possible de sélectionner plus de deux composés en augmentant le nombre de moyens de sélection. Par exemple, en utilisant trois bagues, il est possible de sélectionner trois composés distincts pour distribuer une formulation. Les moyens de sélection peuvent également posséder une position intermédiaire entre plusieurs contenants dans laquelle aucun composé n'est délivré si bien qu'il est possible de ne délivrer qu'un seul composé en positionnant l'un des moyens de sélection dans cette position intermédiaire.

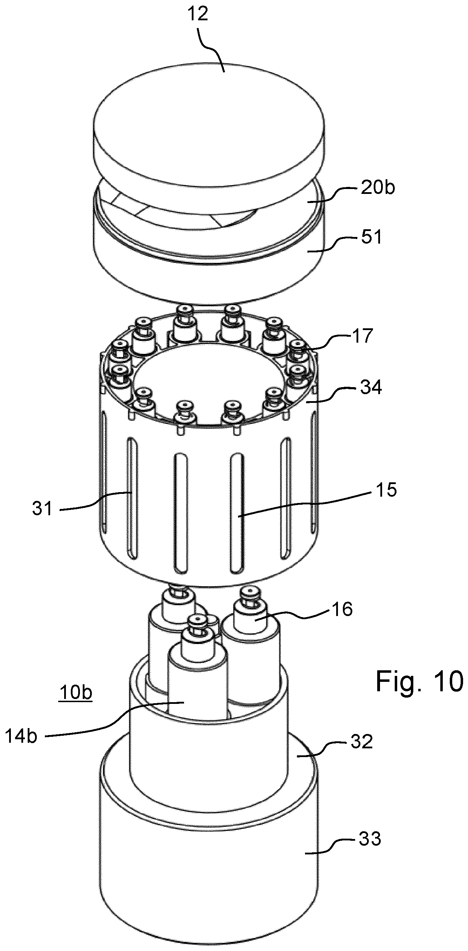

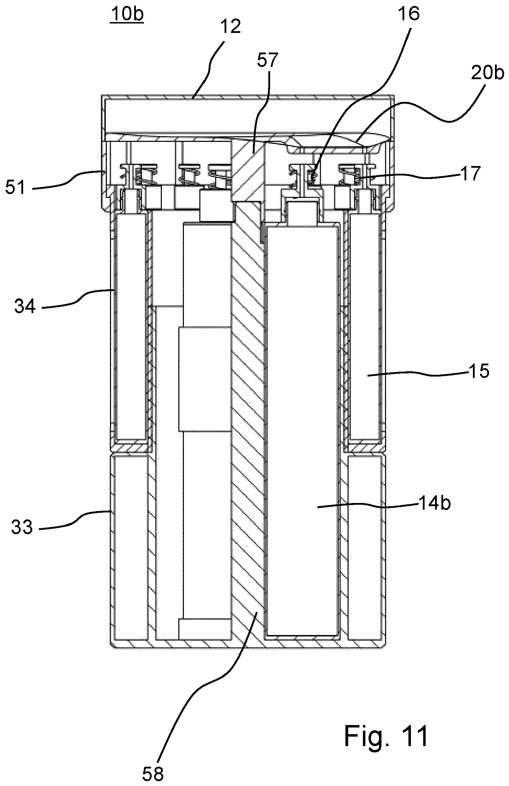

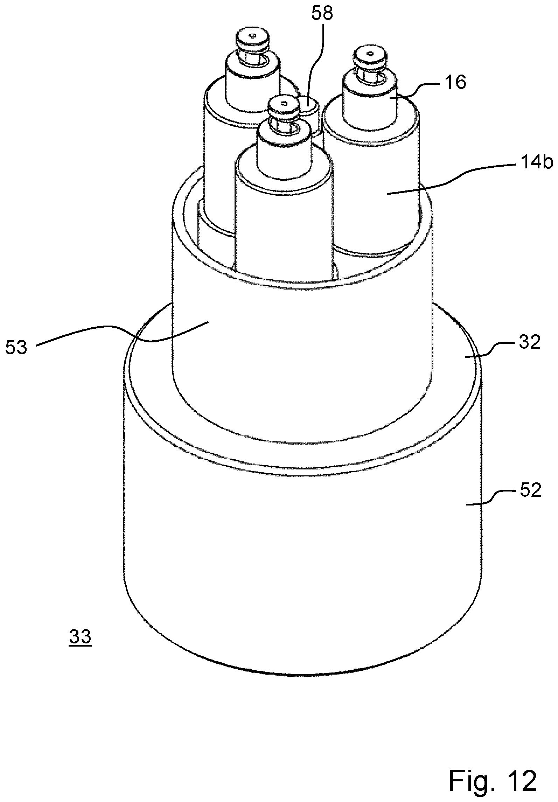

Les figures 9 à 16 illustrent un second mode de réalisation utilisant deux barillets **33-34** pour sélectionner les composés. De la même manière que dans le premier mode de réalisation, une surface de distribution **20b** est disposée en regard d'un ensemble de pompes **16-17** montées sur des contenants **14b-15.** Cependant, cette surface de distribution **20b** est fixe et ce sont les contenants **14b-15** qui sont mobiles par rapport à cette surface de distribution **20b.**

Pour ce faire, le dispositif de distribution **20b** présente un premier barillet **33** comportant un cylindre inférieur **52** et un cylindre supérieur **53** de plus faible diamètre formant un épaulement **32.** Trois contenants **14b** cylindriques sont insérés dans les deux cylindres et dépassent en hauteur.

L'épaulement **32** permet de monter un second barillet **34** prenant la forme d'un cylindre creux supportant douze contenants **15.**

Un bouchon de distribution **51** est monté fixe au-dessus des deux barillets **33-34.** Ce bouchon de distribution **51** présente un axe **57** monté sur un axe central **58** du premier barillet **33** de sorte que l'axe **58** du premier barillet **33** soit mobile en rotation par rapport à l'axe **57** du bouchon de distribution **51.**

Le second barillet **34** est contraint en translation entre l'épaulement **32** et le bouchon de distribution **51** de sorte que ce second barillet **34** soit mobile en rotation autour des axes **57-58,** indépendamment des rotations du premier barillet **33.** Tel qu'illustré sur la figure 14, l'extrémité supérieure du second barillet **33** peut comporter des ergots **54** de sorte à former des détrompeurs de positions par rapport à la surface de distribution **20b.**

Le premier barillet **33** est monté de manière amovible sur l'axe **57** de sorte que les deux barillets puissent être démontés pour remplacer ou remplir un des contenants **14b, 15.** En outre, le niveau des contenants **15** peut être directement visible pour les ouvertures **31.**

Dans le bouchon de distribution **51,** deux orifices **55** et **56** sont ménagés de sorte à permettre aux pompes **16-17** disposées en face de ces orifices **55-56** d'acheminer leurs contenus sur la surface de distribution **20b.** En outre, les orifices **55** et **56** étant fixes, la surface de distribution **20b** peut être structurée pour faciliter la préhension des composés par l'utilisateur, tel qu'illustré sur la figure 16. De la même manière que pour le premier mode de réalisation, l'actionnement des pompes **16-17** peut être réalisé en déplaçant la surface de distribution **20b.**

L'invention permet ainsi d'obtenir un dispositif de distribution d'une formulation d'au moins deux composés choisis parmi un ensemble de composés sélectionnables beaucoup plus transportable que les solutions existantes. En outre, ce dispositif de distribution peut être entièrement mécanique.

Ainsi, un utilisateur peut formuler rapidement et simplement une formulation sur mesure en fonction de ses besoins. Par exemple, avec l'invention, un utilisateur peut formuler une ou plusieurs doses d'une crème cosmétique adaptée pour traiter sa peau.

## Revendications

1. Dispositif de distribution (10b) d'une formulation d'au moins deux composés choisis parmi un ensemble de composés sélectionnables, ledit dispositif comportant :
∘ une pluralité de contenants (14b, 15) stockant lesdits différents composés sélectionnables ;
∘ des moyens de pompage (16-17) associés à chaque contenant (14b, 15) ;
∘ une surface de distribution (20b) comportant au moins deux orifices (55-56) débouchant destinés à acheminer au moins deux composés choisis ;
∘ des moyens de sélection (33-34) permettant de disposer lesdits au moins deux orifices (55-56) débouchant en face des moyens de pompage (16-17) desdits composés choisis de sorte à permettre auxdits moyens de pompage (16-17) desdits composés choisis d'acheminer lesdits composés choisis sur la surface de distribution ; et
∘ un actionneur configuré pour n'activer que les moyens de pompage (16-17) desdits composés choisis et ne délivrer que lesdits au moins deux composés choisis sur ladite surface de distribution (20b).

2. Dispositif de distribution selon la revendication 1, ***dans lequel*** ledit actionneur correspond à un bouton poussoir dont la face supérieure correspond à ladite surface de distribution ( 20b), lesdits moyens de pompage (16-17) ayant une course supérieure ou égale à la course du bouton poussoir.

3. Dispositif de distribution selon la revendication 1 ou 2, ***dans lequel*** au moins un desdits moyens de sélection correspond à un barillet (33-34) intégrant plusieurs contenants (14b, 15), une rotation dudit barillet (33-34) entraînant une modification de la correspondance entre un orifice (55-56) et l'un des contenants (14b, 15) intégré dans ledit barillet (33-34).

4. Dispositif de distribution selon l'une des revendications 1 à 3, ***dans lequel*** ledit dispositif comporte un bouchon de protection amovible (12) configuré pour recouvrir ladite surface de distribution (20b) et inhiber l'activation dudit actionneur.

5. Dispositif de distribution selon l'une des revendications 1 à 4, ***dans lequel*** ledit dispositif comporte deux types de composés distincts stockés dans deux types de contenants distincts (14b, 15) ; lesdits au moins deux composés étant choisis pour obtenir une formulation d'au moins un premier type de composés et d'au moins un second type de composés.

6. Dispositif de distribution selon la revendication 5, ***dans lequel*** des premiers contenants (14b) présentant un volume supérieur à des seconds contenants (15) ; lesdits moyens de pompage (16) associés auxdits premiers contenants (14b) présentent un volume de pompage plus important que lesdits moyens de pompage (17) associés auxdits seconds contenants (15) pour une même activation dudit actionneur.

7. Dispositif de distribution selon la revendication 5 ou 6, ***dans lequel*** ledit dispositif comporte entre un et trois composés du premier type et entre six et huit composés du second type.

8. Dispositif de distribution selon l'une des revendications 5 à 7, ***dans lequel,*** ladite formulation correspondant à une crème cosmétique, ledit premier type de composés correspond à des bases et ledit second type de composés correspond à des sérums.

9. Dispositif de distribution selon l'une des revendications 1 à 8, ***dans lequel,*** ladite pluralité de contenants (14b, 15) est intégrée dans au moins un support (33, 34) présentant au moins deux parties amovibles de sorte à permettre un remplacement ou un remplissage d'au moins un contenant (14b, 15).

## Patentansprüche

1. Vorrichtung zur Verteilung einer Rezeptur aus mindestens zwei Verbindungen, ausgewählt aus verschiedenen auswählbaren Verbindungen, wobei diese Vorrichtung umfasst:
- verschiedene Behälter (14b, 15) zur Aufbewahrung dieser verschiedenen auswählbaren Verbindungen;
- Pumpvorrichtungen (16-17), verbunden mit jedem Behälter (14b, 15);
- eine Verteilerfläche (20b), die mindestens zwei einmündende Öffnungen (55-56) enthält, die dazu bestimmt sind, mindestens zwei ausgewählte Verbindungen zuzuführen;
- Auswahlvorrichtungen (33-34), mit denen es möglich ist, diese mindestens zwei einmündenden Öffnungen (55-56) vor die Pumpvorrichtungen (16-17) für die ausgewählten Verbindungen zu bringen, so dass diese Pumpvorrichtungen (16-17) dieser ausgewählten Verbindungen in der Lage sind, diese ausgewählten Verbindungen auf die Verteilerfläche zu bringen; und
- einen Schalter, dazu konfiguriert, nur die Pumpvorrichtungen (16-17) der erwähnten Verbindungen zu aktivieren und diese mindestens zwei Verbindungen auf diese Verteilerfläche (20b) zu bringen.

2. Verteilervorrichtung nach Anspruch 1, ***bei der*** es sich bei dem erwähnten Schalter um einen Drucktaster handelt, dessen obere Fläche dieser Verteilerfläche (20b) entspricht, diese Pumpvorrichtungen (16-17) haben einen Hubweg größer oder gleich dem Hubweg des Drucktasters.

3. Verteilervorrichtung nach Anspruch 1 oder 2, ***bei der*** mindestens eins dieser Mittel zur Auswahl einer Trommel (33-34) entspricht, die mehrere Behälter (14b, 15) enthält, eine Drehung dieser Trommel (33-34) führt zu einer Änderung der Übereinstimmung zwischen einer Öffnung (55-56) und dem in dieser Trommel (33-34) integrierten Behälter (14b, 15).

4. Verteilervorrichtung nach einem der Ansprüche 1 bis 3, ***bei der*** diese Vorrichtung einen abnehmbaren Schutzstopfen (12) enthält, konfiguriert zur Abdeckung der erwähnten Verteilerfläche (20b) und zur Vermeidung einer Aktivierung dieses Schalters.

5. Verteilervorrichtung nach einem der Ansprüche 1 bis 4, ***bei der*** diese Vorrichtung zwei verschiedene Typen Verbindungen enthält, die in zwei verschiedenen Behältertypen (14b, 15) untergebracht sind; diese mindestens zwei Verbindung werden so ausgewählt, dass sich eine Rezeptur mindestens eines ersten Verbindungstyps und mindestens eines zweiten Verbindungstyps ergibt.

6. Verteilervorrichtung nach Anspruch 5, ***bei der*** die ersten Behälter (14b) ein Volumen aufweisen, dass größer ist, als das der zweiten Behälter (15); die erwähnten, mit diesen ersten Behältern (14b) kombinierten Pumpvorrichtungen (16) weisen ein höheres Pumpvolumen auf, als die erwähnten, mit diesen zweiten Behältern (15) kombinierten Pumpvorrichtungen (17) bei derselben Betätigung des erwähnten Schalters.

7. Verteilervorrichtung nach Anspruch 5 oder 6, ***bei der*** diese Vorrichtung zwischen einer und drei Verbindungen des ersten Typs und zwischen sechs und acht Verbindungen des zweiten Typs enthält.

8. Verteilervorrichtung nach einem der Ansprüche 5 bis 7, ***bei der*** die erwähnte Rezeptur einer kosmetischen Creme entspricht, der erwähnte erste Typ Verbindungen entspricht Grundsubstanzen und der erwähnte zweite Typ Verbindungen entspricht Seren.

9. Verteilervorrichtung nach einem der Ansprüche 1 bis 8, ***bei der*** diese verschiedenen Behälter (14b, 15) in mindestens einem Halter (33, 34) integriert sind, der mindestens zwei abnehmbare Teile aufweist, so dass mindestens ein Behälter (14b, 15) ausgetauscht oder gefüllt werden kann.

## Claims

1. A device (10b) for dispensing a formulation of at least two compounds selected from a set of selectable compounds, said device comprising:
- a plurality of containers (14b, 15) storing the different selectable compounds;
- pumping means (16-17) associated with each container (14b, 15);
- a dispensing surface (20b) comprising at least two through openings (55-56) intended to deliver at least two selected compounds;
- selection means (33-34) enabling the positioning of said at least two openings (55-56) in front of the pumping means (16-17) of said chosen compounds in such a way as to allow said pumping means (16-17) of said chosen compounds to convey said chosen compounds onto the dispensing surface (20b); and
- an actuator configured to activate only the pumping means (16-17) of said chosen compounds and to only deliver said at least two selected compounds onto said dispensing surface (20b).

2. Dispensing device according to claim 1, ***wherein*** said actuator corresponds to a push button having its upper surface corresponding to said dispensing surface (20b), said pumping means (16-17) having a stroke greater than or equal to the stroke of the push button.

3. Dispensing device according to claim 1 or 2, ***wherein*** at least one of said selection means corresponds to a barrel (33-34) integrating a plurality of containers (14b, 15), a rotation of said barrel (33-34) causing a modification of the matching between an opening (55-56) and one of the containers (14b, 15) integrated in said barrel (33-34).

4. Dispensing device according to any of claims 1 to 3, ***wherein*** said device comprises a removable protection cap (12) configured to cover said dispensing surface (20b) and inhibit the activation of said actuator.

5. Dispensing device according to any of claims 1 to 4, ***wherein*** said device comprises two different types of compounds stored in two different types of containers (14b, 15); said at least two compounds being selected to obtain a formulation of at least a first type of compounds and of at least a second type of compounds.

6. Dispensing device according to claim 5, ***wherein*** first containers (14b) have a greater volume than second containers (15); said pumping means (16) associated with said first containers (14b) have a much larger pumping volume than said pumping means (17) associated with said second containers (15) for a same activation of said actuator.

7. Dispensing device according to claim 5 or 6, ***wherein*** said device comprises between one and three compounds of the first type and between six and eight compounds of the second type.

8. Dispensing device according to any of claims 5 to 7, ***wherein*** said formulation corresponding to a cosmetic cream, said first type of compounds corresponds to bases and said second type of compounds corresponds to serums.

9. Dispensing device according to any of claims 1 to 8, ***wherein*** said plurality of containers (14b, 15) is integrated in at least one support (33, 34) having at least two removable portions to enable to replace or to fill at least one container 14b, 15).
